Europäisches Patentamt

European Patent Office

Office européen des brevets

(11) Veröffentlichungsnummer: **0 445 786 A1**

# EUROPÄISCHE PATENTANMELDUNG

(21) Anmeldenummer: 91103432.0

(22) Anmeldetag: 06.03.91

(51) Int. Cl.5: **F25C 1/00, C12N 1/04, A23L 3/36**

(30) Priorität: 07.03.90 DE 4007164

(43) Veröffentlichungstag der Anmeldung:
11.09.91 Patentblatt 91/37

(84) Benannte Vertragsstaaten:
AT CH DE FR GB IT LI NL

(71) Anmelder: **Linde Aktiengesellschaft**
**Abraham-Lincoln-Strasse 21**
**W-6200 Wiesbaden(DE)**

(72) Erfinder: **Sonnentag, Michael, Dipl.-Ing.**
**Hofmannstr. 19**
**W-8000 München 70(DE)**
Erfinder: **Schneeberger, Heribert, Dipl.-Ing.**
**Grünwalder Str. 46**
**W-8000 München 46(DE)**

(74) Vertreter: **Schaefer, Gerhard, Dr.**
**Linde Aktiengesellschaft Zentrale**
**Patentabteilung**
**W-8023 Höllriegelskreuth(DE)**

(54) Verfahren und Vorrichtung zum Gefrieren von fliessfähiger Materie.

(57) Die Erfindung betrifft ein Verfahren und eine Vorrichtung zum Gefrieren fließfähiger Materie, wie z.B. Bakteriensuspensionen. Die fließfähige Materie wird mittels einer Zweistromdüse 3 auf eine in einem Bad 4 befindliche kryogene Flüssigkeit aufgesprüht. Die Zweistromdüse 3 wird hierzu einerseits mit der fließfähigen Materie und andererseits mit einem unter Druck stehenden Treibgas beaufschlagt. Vorzugsweise ist auch die fließfähige Materie druckbeaufschlagt. Durch Veränderung des Drucks des Treibgases und/oder des Drucks der fließfähigen Materie ist ein sehr weiter Bereich von gefrorenen Pulvern bis Pellets herstellbar.

EP 0 445 786 A1

Die Erfindung betrifft ein Verfahren zum Gefrieren fließfähiger Materie mittels einer kryogenen Flüssigkeit.

Es besteht oft aus den verschiedensten Gründen die Notwendigkeit, fließfähige Materie zu gefrieren. Beispielsweise werden Suspensionen von lebendem Zellmaterial durch Gefrieren mit einer kryogenen Flüssigkeit konserviert, um dann in der Lebensmittelindustrie oder chemisch-pharmaceutischen Industrie verwendet zu werden. Ein Beispiel dafür sind Bakteriensuspensionen, die als Bakterienmischkultur zur Joghurtherstellung oder Käsezubereitung eingesetzt werden. Bei derartigen empfindlichen Materialien muß das Einfrieren möglichst schnell und gleichmäßig erfolgen, um Kälteschäden zu vermeiden. Durch unkontrolliertes Einfrieren kann infolge starker Eiskristallbildung die Bakterienzellwand zerstört werden. Das Gefrieren fließfähiger Materie mittels einer kryogenen Flüssigkeit wird auch in der chemischen Industrie verwendet, um beispielsweise durch rasches Abkühlen eine chemische Reaktion zu stoppen.

In der DE-OS 37 11 169 ist z.B. eine Vorrichtung zum kontrollierten Einfrieren von zähfließenden Flüssigkeiten bekannt. Die zähfließende Flüssigkeit, beispielsweise eine Bakteriensuspension oder ein Impfserum, wird aus einem Vorratsbehälter, der einen Lochboden aufweist, in ein Bad einer kryogenen Flüssigkeit getropft. Zähigkeit der Flüssigkeit und Durchmesser der Bohrungen im Lochboden bestimmen die Tropfengröße. Diese Anordnung ermöglicht es jedoch nicht, sehr feine Pulver zu erzeugen oder die Partikelgröße der gefrorenen Tropfen ohne Änderung der Viskosität der zugefrierenden zähfließenden Flüssigkeit zu verändern.

Der vorliegenden Erfindung liegt die Aufgabe zugrunde, ein Verfahren der eingangs genannten Art sowie eine Vorrichtung zur Durchführung des Verfahrens so auszugestalten, daß sehr feine Pulver der gefrorenen Materie erzeugt werden können und die Partikelgrößen der gefrorenen Materie leicht verändert werden können.

Diese Aufgabe wird erfindungsgemäß dadurch gelöst, daß die fließfähige Materie mittels einer Düse auf die kryogene Flüssigkeit aufgesprüht wird.

Mit Vorteil wird dafür eine Zweistromdüse verwendet, die einerseits mit der fließfähigen Materie und andererseits mit einem unter Druck stehenden Treibgas beaufschlagt wird. Unter einer Zweistromdüse ist eine Düse zu verstehen, die einen inneren Düsenkörper aufweist, der konzentrisch von einem äußeren Düsenkörper umgeben ist. Vorzugsweise wird der innere Düsenkörper mit der fließfähigen Materie beaufschlagt, während der äußere Düsenkörper mit dem Treibgas druckbeaufschlagt wird. In dem durch das Treibgas hervorgerufenen Austrittsstrahl wird die fließfähige Materie mitgerissen.

Es ergibt sich so ein Strahl von in Tropfen zerteilter fließfähiger Materie, die auf die Flüssigkeitsoberfläche einer beispielsweise in einem Bad befindlichen kryogenen Flüssigkeit gerichtet ist. Die Tropfengröße kann durch Variation der Drücke von fließfähiger Materie und/oder Treibgas variiert werden.

Als Treibgas wird vorzugsweise ein gegenüber der fließfähigen Materie inertes Gas verwendet. Dies ist insbesondere beim Gefrieren von biologischen Suspensionen zu empfehlen, um eine Veränderung der Suspension z.B. infolge von Oxidationsreaktionen zu vermeiden. Soll ein Material gefroren werden, das gegenüber Oxidationsreaktionen unempfindlich ist, so kann beispielsweise auch Druckluft als Treibgas verwendet werden.

Entsprechend einer anderen Ausführungsform der Erfindung ist vorgesehen, daß die fließfähige Materie mittels einer Düse in einem Strahl auf die kryogene Flüssigkeit aufgesprüht wird, wobei mittels mindestens einer weiteren Düse ein Gasstrahl auf den Strahl der fließfähigen Materie gerichtet wird, um diesen zu zerstreuen.

Vorteilhafterweise wird als kryogene Flüssigkeit flüssiger Stickstoff eingesetzt. Es ist außerdem zweckmäßig, als Treibgas gasförmigen Stickstoff zu verwenden. Dies hat den Vorteil, daß das Gefrieren unter Inertbedingungen abläuft und darüber hinaus nur ein Flüssigstickstoffbehälter notwendig ist, der einerseits den flüssigen Stickstoff für das kryogene Bad und andererseits den gasförmigen Stickstoff für die Verwendung als Treibgas liefert.

Gemäß einer Weiterbildung des Erfindungsgedankens ist vorgesehen, außer dem Treibgas auch die fließfähige Materie mit Druck zu beaufschlagen. Dies ist insbesondere bei zähfließender Materie zu empfehlen, um einen gleichmäßigen Strahl der fließfähigen Materie zu erhalten.

Mit der Erfindung ist eine Möglichkeit geschaffen worden, die Partikelgrößen der gefrorenen Materie schnell und bequem zu ändern, ohne auf bewegliche Teile angewiesen zu sein oder auf eine Veränderung der Zusammensetzung und/oder Viskosität der fließfähigen Materie zurückgreifen zu müssen. Die Partikelgröße der gefrorenen Materie kann einfach durch Variation des Drucks des Treibgases und/oder der fließfähigen Materie verändert werden. Erhöht man den Druck des Treibgases im äußeren Düsenkörper der Zweistromdüse, so ergibt sich ein breit aufgefächerter Strahl der fließfähigen Materie. Infolgedessen tritt die fließfähige Materie sehr fein verteilt auf der Oberfläche der kryogenen Flüssigkeit auf und wird dementsprechend zu kleinen Partikeln gefroren. Wird andererseits der Druck der fließfähigen Materie im inneren Düsenkörper der Zweistromdüse erhöht, so wird der Strahl stärker fokussiert, was zu größeren Partikeln führt. Außerdem kann die Partikelgröße durch die Wahl der geometrischen Form der Zweistromdüse verändert werden. Insgesamt ist mit der Erfindung ein sehr weiter Bereich von Pulvern bis Pellets herstellbar. Sehr feine Pulver von gefrorener Materie werden beispielsweise bei biologischen Zellsuspensionen gefordert, um einen Aufschluß der Zellen zu erleichtern. Vielfach ist nämlich der Zellinhalt, z.B. Enzyme, Stoffwechselprodukte etc., für die Lebensmittelindustrie oder chemisch-pharmazeutische Industrie von besonderem Interesse. Andererseits können in bestimmten Anwendungsfällen auch größere Partikel gefordert sein, um beispielsweise eine zu starke Staubentwicklung zu verhindern oder leicht dosierbare Präparate zu erhalten.

Das Bad der kryogenen Flüssigkeit kann beispielsweise als Dewar oder als Tauchfroster ausgebildet sein. Im ersten Fall werden die gefrorenen Partikel aus der kryogenen Flüssigkeit abgeschöpft oder mit der kryogenen Flüssigkeit aus dem Bad abgelassen und dann gegebenenfalls einer Weiterverarbeitung zugeführt. Im zweiten Fall befördert ein im Bad unterhalb der Flüssigkeitsoberfläche der kryogenen Flüssigkeit verlaufendes Förderband die gefrorenen Partikel kontinuierlich aus dem Bad heraus und führt sie beispielsweise einer Weiterverarbeitung zu.

Insgesamt bietet die Erfindung den entscheidenden Vorteil, daß eine feinkörnige gleichmäßige und leicht in der Partikelgröße veränderbare gefrorene Materie erhalten wird, die für eine Weiterverarbeitung, wie z.B. einem Zellaufschluß von biologischen Zellen, bestens geeignet ist. Außerdem werden Verklumpungen im Bad zuverlässig verhindert.

Die Erfindung eignet sich ganz allgemein zum Gefrieren fließfähiger Materie jeder Art. Insbesondere ist sie für das Einfrieren von biologischen Zellsuspensionen geeignet, die z.B. als Starterkulturen in der Fermentationstechnik oder nach Zellaufschluß zur Enzymgewinnung verwendet werden. Darüber hinaus kann die Erfindung in der chemischen Industrie eingesetzt werden, um z.B. durch rasche Abkühlung von Stoffen eine chemische Reaktion zu stoppen.

Eine Vorrichtung zur Durchführung des Verfahrens besteht aus einem Bad, das eine kryogene Flüssigkeit enthält. Erfindungsgemäß ist außerhalb der kryogenen Flüssigkeit eine Düse für die fließfähige Materie so angeordnet, daß ihre Austrittsöffnung auf die Flüssigkeitsoberfläche der kryogenen Flüssigkeit gerichtet ist.

In einer besonder bevorzugten Ausführungsform der Erfindung ist die Düse als Zweistromdüse ausgebildet, die eine Zufuhrleitung für die fließfähige Materie und eine weitere Zufuhrleitung für das unter Druck stehende Treibgas aufweist. Zweckmäßigerweise steht die Zufuhrleitung für die fließfähige Materie mit dem inneren Düsenkörper der Zweistromdüse in Verbindung und die Zufuhrleitung für das unter Druck stehende Treibgas mit dem äußeren Düsenkörper.

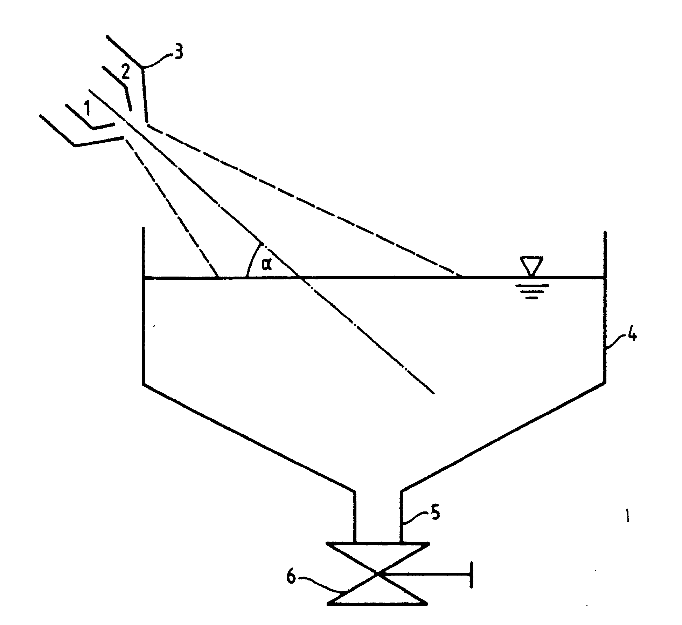

Im folgenden soll die Erfindung anhand eines in der Zeichnung schematisch dargestellten Ausführungsbeispiels näher erläutert werden.

Die Figur zeigt eine Anlage zum Gefrieren von biologischen Zellsuspensionen.

In einem Bad 4 befindet sich flüssiger Stickstoff, auf den mittels einer außerhalb des Bades 4 angebrachten Zweistromdüse 3 eine biologische Zellsuspension, eine sogenannte Kulturbrühe, aufgesprüht wird. Dem inneren Düsenkörper 1 der Zweistromdüse 3 wird die Kulturbrühe zugeführt, während der äußere Düsenkörper 2 mit unter Druck stehendem gasförmigen Stickstoff beaufschlagt wird, der als Treibgas wirkt. Um einen besonders gleichmäßigen Strahl der Kulturbrühe zu erreichen, wird auch die Kulturbrühe druckbeaufschlagt. Durch Veränderung des Drucks der Kulturbrühe und/oder des gasförmigen Stickstoffs kann eine bestimmte Körnung der gefrorenen Kulturbrühe erreicht werden. Erhöht man den Druck des gasförmigen Stickstoffs im äußeren Düsenkörper 2, so erhält man einen breit aufgefächerten Strahl, so daß die Kulturbrühe fein verteilt auf der Flüssigkeitsoberfläche des im Bad 4 befindlichen flüssigen Stickstoffs auftrifft und als feines Pulver einfriert. Aufgrund der sehr geringen Partikelgrößen des Pulvers erfolgt der Einfriervorgang sehr schnell von außen nach innen, so daß keine Zeit für die Ausbildung großer Eiskristalle bleibt. Die auf diese Weise eingefrorene Kulturbrühe zeichnet sich deshalb durch eine besonders hohe Überlebensrate der Zellen aus. Wird dagegen der Druck der Kulturbrühe im inneren Düsenkörper 1 erhöht, so erhält man einen fokussierten Strahl und infolge dessen eine grobkörnige gefrorene Kulturbrühe. Die Zweistromdüse 3 ist um einen Winkel α gegen die Horizontale geneigt angeordnet. Durch Schwenken der Zweistromdüse 3 kann der Winkel α, unter dem die Kulturbrühe auf die Flüssigkeitsoberfläche des flüssigen Stickstoffs auftrifft, verändert werden. Auf diese Weise kann ebenfalls die Körnung der gefrorenen Kulturbrühe beeinflußt werden.

Die gefrorene Kulturbrühe wird über einen mit einem Ventil 6 versehenen Ablauf 5 aus dem Bad 4 abgezogen und unter Inertbedingungen in Vorratsbehälter abgepackt, um bei Bedarf z.B. als Starterkultur bei Fermentationsprozessen eingesetzt zu werden.

**Patentansprüche**

1. Verfahren zum Gefrieren fließfähiger Materie mittels einer kryogenen Flüssigkeit, dadurch gekennzeichnet, daß die fließfähige Materie mittels einer Düse auf die kryogene Flüssigkeit aufgesprüht wird.

2. Verfahren nach Anspruch 1, dadurch gekennzeichnet, daß als Düse eine Zweistromdüse verwendet wird, die einerseits mit der fließfähigen Materie und andererseits mit einem unter Druck stehenden Treibgas beaufschlagt wird.

3. Verfahren nach Anspruch 1 oder 2, dadurch gekennzeichnet, daß als Treibgas ein Inertgas, vorzugsweise gasförmiger Stickstoff, verwendet wird.

4. Verfahren nach einem der Ansprüche 1 bis 3, dadurch gekennzeichnet, daß als kryogene Flüssigkeit flüssiger Stickstoff verwendet wird.

5. Verfahren nach einem der Ansprüche 1 bis 4, dadurch gekennzeichnet, daß die fließfähige Materie druckbeaufschlagt wird.

6. Verfahren nach einem der Ansprüche 1 bis 5, dadurch gekennzeichnet, daß als fließfähige Materie eine Zellsuspension verwendet wird.

7. Verfahren nach einem der Ansprüche 1 bis 6, dadurch gekennzeichnet, daß in Abhängigkeit von gewünschter Partikelgröße der gefrorenen Materie der Druck der fließfähigen Materie und/oder des Treibgases verändert wird.

8. Vorrichtung zur Durchführung des Verfahrens nach einem der Ansprüche 1 bis 7 mit einem Bad, das eine kryogene Flüssigkeit enthält, dadurch gekennzeichnet, daß außerhalb der kryogenen Flüssigkeit eine mit einer Austrittsöffnung auf die Flüssigkeitsoberfläche gerichtete Düse angeordnet ist.

9. Vorrichtung nach Anspruch 8, dadurch gekennzeichnet, daß die Düse als Zweistromdüse ausgebildet ist, die eine Zufuhrleitung für die fließfähige Materie und eine Zufuhrleitung für das unter Druck stehende Treibgas aufweist.

**Europäisches Patentamt**

# EUROPÄISCHER RECHERCHENBERICHT

Nummer der Anmeldung

# EP 91 10 3432

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | WO-A-8 809 470   (ROSENMUND) <br> * Seite 3, letzter Absatz - Seite 6, letzter Absatz; Figuren 1-2 * | 1-9 | F 25 C 1/00 <br> C 12 N 1/04 <br> A 23 L 3/36 |
| Y | EP-A-0 225 081   (TAIYO SANSO) <br> * Seite 4, Zeile 1 - Seite 9, Zeile 40; Figuren 1-15 * | 1-9 | |
| A | EP-A-0 266 859   (TAIYO SANSO) <br> * Spalte 6, Zeile 37 - Spalte 15, Zeile 41; Figuren 1-10 * | 1-4 | |
| A | DE-A-2 901 884   (BAXTER TRAVENOL LABORATORIES) <br> * Seite 11, Zeile 26 - Seite 20, Zeile 36; Figuren 1-2 * | 1,4-6,8 | |
| A | US-A-4 655 047   (TEMPLE) <br> * Spalte 4, Zeile 15 - Spalte 6, Zeile 16; Figuren 1-2 * | 1,4,6,8 | |
| A | US-A-2 083 072   (LINDSEY) | | |
| A | US-A-3 721 725   (BRIGGS) | | |
| A | US-A-4 848 094   (DAVIS) | | RECHERCHIERTE SACHGEBIETE (Int. Cl.5) |
| A | EP-A-0 306 132   (CELL SYSTEMS) | | F 25 C <br> C 12 N <br> A 23 L |
| A | FR-A-2 203 591   (VSESOJUZNY NAUCHNO-ISSLEDOVATELSKY INSTITUT MASLODELNOI I SYRO-DELNOI PROMYSHLENNOSTI) | | |

Der vorliegende Recherchenbericht wurde für alle Patentansprüche erstellt

| Recherchenort | Abschlußdatum der Recherche | Prüfer |
|---|---|---|
| Den Haag | 14 Juni 91 | BOETS A.F.J. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument
-------------------------------------------------
&: Mitglied der gleichen Patentfamilie, übereinstimmendes Dokument